# EUROPEAN PATENT SPECIFICATION

(11) **EP 3 687 971 B1**
(45) Date of publication and mention of the grant of the patent: **23.02.2022**
(21) Application number: 17791757.2
(22) Date of filing: 28.09.2017
(51) Int. Cl.: C07C 231/24

(54) **METHODS FOR PROVIDING HIGHLY PURE PALMITOYLETHANOLAMIDE (PEA)**
VERFAHREN ZUR HERSTELLUNG VON HOCHREINEM PALMITOYLETHANOLAMID (PEA)
PROCÉDÉS PERMETTANT DE FOURNIR UNE PALMITOYLÉTHANOLAMIDE (PEA) TRÈS PURE

(43) Date of publication of application: 05.08.2020
(73) Proprietor: Innexus Nutraceuticals B.V., 6524 DA Nijmegen (NL)
(72) Inventor: KELLER, Erik, 9774 PX Adorp (NL); ZONNEVELD, Samuel Johannes Dabbous, 6524 DA Nijmegen (NL)
(74) Representative: V.O.
(86) International application number: PCT/NL2017/050647
(87) International publication number: WO 2019/066644

(56) References cited:
- WO-A1-2006/109321
- US-A1- 2013 303 795

## Description

The invention relates to the field of medicinal chemistry. More in particular, it relates to methods for providing highly pure, pharmaceutical grade synthetic Palmitoylethanolamide (PEA), to pure PEA preparation obtainable thereby, and the uses thereof.

Palmitoylethanolamide (N-(2-hydroxyethyl)hexadecanamide, PEA) (CAS 544-31-0; EINECS 208-867-9), also known as palmitoylethanolamide or palmidrol, is a naturally occurring C16:0 fatty acid derivative wherein the carboxylate function is amidated by the primary amine of ethanolamine. N-acylethanolamine compounds are known to have anti-inflammatory and anti-nociceptive effects, as described in Lambert *et al.* (Lambert DM, Vandevoorde S, Jonsson KO, Fowler CJ. Curr Med Chem. 2002;9:663-74); Lambert DM *et al.* (Lambert DM, DiPaolo FG, Sonveaux P, Kanyonyo M, Govaerts SJ, Hermans E, Bueb J, Delzenne NM, Tschirhart EJ. Biochim Biophys Acta. 1999;1440:266-74); Brown AJ (Br J Pharmacol. 2007;152(5):567-75); and US 5,506,224 and US 2005/0054730.

A large number of scientific investigations on the effects of PEA and PEA-related compounds are available, and give rise to new therapeutic opportunities. Multiple synthetic procedures of PEA have been reported. PEA can for example be prepared via the coupling of palmitic acid and ethanolamine in the presence of a coupling reagent. Exemplary coupling routes for synthesis of PEA involve the use of 1-Ethyl-3-(3-dimethylaminopropyl)carbodiimide (EDCI) in the presence of 4-dimethylaminopyridine (DMAP) in methylene chloride (see WO2015/179190), or 1-Cyano-2-ethoxy-2-oxoethylidenaminooxy)dimethylamino-morpholino-carbenium hexafluorophosphate (COMU) in the presence of Di-isopropylethylamine (DIPEA) in a mixture of methylene chloride and acetonitrile *(*Chemistry and physics or lipids 2012, 165, 705). Still further examples involve 2-(1H-Benzotriazole-1-yl)-1,1,3,3-tetramethylaminium tetrafluoroborate (TBTU) in the presence of triethylamine in acetonitrile *(*Bioorganic and Medicinal Chemistry 2011 , 19, 1520), *N,N'*-carbodiimide (CDI) in methylene chloride *(*Tetrahedron Letters 1980, 21, 841) or *N,N'*-dicyclohexylcarbodiimide (DCC) in the presence or DMAP in methylene chloride *(*Chemistry Letters; (1985); p. 701). The synthesis of PEA typically involves aqueous workup and purification using flash chromatography, often in combination with crystallization.

Alternatively, PEA can be prepared by reacting palmitic acid with acetic anhydride followed by ethanolamine *(*J. Am. Chem. Soc., 1952, 74 (13), 3442), or via aminolysis of isopropyl palmitate and ethanolamine in the presence of sodium ethoxide/ethanol (see US2016/038443), via aminolysis of ethyl, methyl, or vinyl palmitate or the reaction of palmitoyl chloride and ethanolamine in the presence of a base. Moreover, synthesis of PEA has been reported using activation of palmitic acid via the formation of an active ester *(*Archiv der Pharmazie 342 (2009) 34; WO2006/109321; Chemistry Letters; (1985) 701; Chemical & Pharmaceutical Bulletin 32 (1984) 2687).

A procedure that is frequently used for large scale preparation of PEA for pharmaceutical use involves the direct heating of the palmitic acid in the presence of ethanolamine, followed by removal of excess of ethanolamine to provide a crude PEA preparation.

It is known that crude PEA prepared from direct reaction of palmitic acid with ethanolamine is often contaminated with a number of impurities. The level of impurities can be reduced by PEA crystallization from a solvent, such as methanol. However, it was consistently observed by the present inventors that efficient removal of impurities to an acceptable level while maintaining acceptable yields failed.

Therefore, the present inventors aimed at minimizing such impurities in crude PEA prepared from direct reaction of palmitic acid with ethanolamine. To that end, they first set out to chemically characterize the contaminants, and thereafter provide a method to minimize their presence.

Using HPLC-MS techniques, it was observed that crude PEA contains several contaminants. These were identified as N-(2-(2-aminoethoxy)ethyl)palmitamide, Myristoylethanolamide, Stearoylethanolamide, *N,N'*-(oxybis(ethane-2,1-diyl))dipalmitamide and 2-Palmitamidoethyl palmitate. All impurities could be attributed either to those resulting from impurities in the starting material or to unwanted side reactions.

It was surprisingly found that heating a suspension of crude PEA in a solvent mixture in the presence of a strong base catalyst to reflux, thereby allowing the formation of crystalline PEA, is highly effective to isolate crystalline PEA that is essentially free from impurities which remain in solution. Without wishing to be bound be any theory, it is envisioned that the presence of the more labile ester functionality in PEAP compared to the amide functionality aids in the removal of PEAP from the crude product. By adding an base catalyst as additive to the crystallization medium to facilitate solvolysis of the ester functionality, PEAP could completely be eliminated to provide PEA and methyl palmitate. After crystallization, methyl palmitate remains in solution and PEA free from unwanted impurities is obtained.

Herewith, the invention provides a method for providing pure palmitoylethanolamide (PEA), comprising the steps of (i) heating a suspension of crude PEA in a solvent mixture comprising an alcohol to reflux in the presence of a base catalyst having a pKa (in water) of at least 13, thereby allowing the formation of crystalline PEA; and (ii) isolating said crystalline PEA.

A schematic outline of the synthesis and subsequent refining of PEA according to the invention is exemplified in Scheme 1.

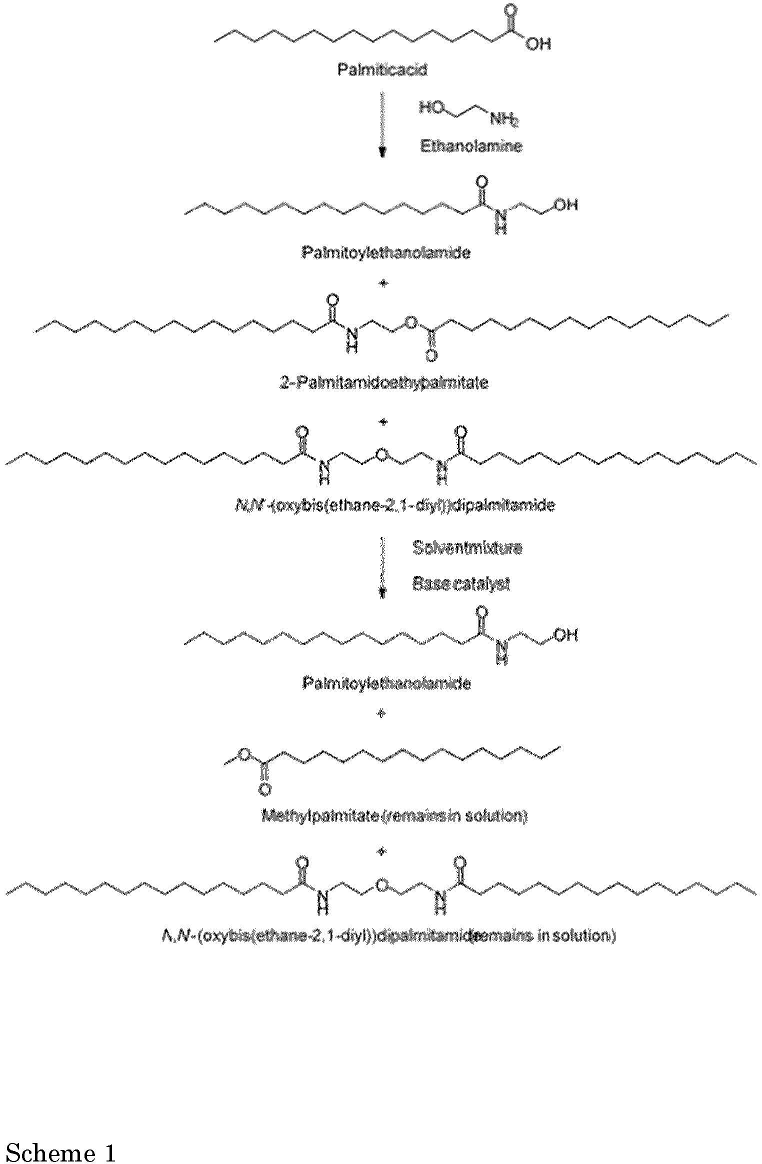

The first step in a method of the invention comprises preparing a suspension of crude PEA in a suitable solvent mixture. Typically, a method of the invention finds its use for purifying crude PEA that is obtained from a direct reaction of palmitic acid and ethanolamine. For example, the crude PEA for use as starting material in a method provided herein comprises one or more unwanted substances selected from the group consisting of N-(2-(2-aminoethoxy)ethyl)palmitamide, Myristoylethanolamide, Stearoylethanolamide, *N,N'*-(oxybis(ethane-2,1-diyl))dipalmitamide and 2-Palmitamidoethyl palmitate. The suspension may comprise about 0.1- 1 kg per liter, preferably 0.2 to 0.4 kilogram of PEA per liter of solvent mixture.

The solvent mixture preferably comprises an aliphatic alcohol, more preferably a C₁-C₃ alcohol, in combination with a suitable co-solvent. Preferred co-solvents include aromatic hydrocarbons like toluene, mesitylene, benzene, xylene and ethylbenzene. Good results were obtained using toluene as co-solvent. In a preferred embodiment, the solvent mixture comprises or consists of a combination of toluene and an alcohol, preferably toluene and methanol or toluene and ethanol. For example, the solvent mixture consists of toluene and methanol in a relative ratio in the range of 1.4 : 1 to 1.8 :1, preferably 1.5:1 to 1.7:1, by volume.

The suspension of crude PEA is heated, preferably while stirring, to reflux in the presence of a strong base catalyst. In one embodiment, the base catalyst has a pKa (in H₂O) of at least 13.5, preferably at least 14.

Good results are obtained wherein the base catalyst is an amidine base or a strong guanidine base. Such base catalysts are known in the art, see for example "Superbases for Organic Synthesis: Guanidines, Amidines, Phosphazenes and Related Organocatalysts, Amidines" in Organic Synthesis 50-91 .

In one embodiment, the base catalyst is an amidine base. Amidines are strong bases (pKa ranges from 5-12). The protonation occurs on the imino nitrogen leading to symmetrical amidinium ion which is stabilized by resonance as in the isoelectronic carboxylate anion. Preferably, the amidine base for use in a method of the invention is selected from the group consisting of 1,8-diazabicyclo[5.4.0]undec-7-ene (DBU), 1,5-diazabicyclo[4.3.0]non-5-ene (DBN), 2,3,4,6,7,8,9,10-octahydropyrimido[1,2-a]azepine), NaHCO₃ and NaOCH₃.

In another embodiment, the base catalyst is a strong guanidine base, preferably triazabicyclodecene (1,5,7-triazabicyclo[4.4.0]dec-5-ene; TBD).

The reaction is allowed to proceed for a time period sufficient to achieve solvolysis of the impuritie(s), typically at least 0.5 hour, preferably at least 1 hour. In one embodiment, the suspension is heated to reflux for about 1-2 hours. Thereafter, the reaction mixture is allowed to cool to room temperature, resulting in the formation of pure, crystalline PEA. The crystalline PEA is suitably isolated by methods known in the art, e.g. comprising the filtering and washing crystalline PEA.

Also disclosed herein is a pure PEA preparation comprising at least 98%, preferably at least 99%, more preferably at least 99.5 % by weight PEA, obtainable by a method according to the invention. This PEA preparation is typically characterized by being essentially devoid (e.g. less than 2%, preferably less than 1.5%, more preferably less than 1% by weight in total) of unwanted substances selected from the group consisting of N-(2-(2-aminoethoxy)ethyl)palmitamide Myristoylethanolamide, Stearoylethanolamide, *N,N'*-(oxybis(ethane-2,1-diyl))dipalmitamide and 2-Palmitamidoethyl palmitate (PAEP).

A pure PEA preparation is advantageously used as human or veterinary supplement or medicament. Therapeutic applications of PEA include those already known in the art, as well as any application yet to be discovered. For example, disclosed herein is the a pure PEA preparation for use in a method of reducing or preventing pain in a mammalian subject, preferably chronic pain, neuropathic pain or a related disorder based on overactivation of glia and glia-related cells, such as in diabetes and glaucoma. Also disclosed is a pure PEA preparation for use in a method of reducing or preventing symptoms associated with influenza, common cold, infections, auto-immune disease, atopic or contact dermatitis, depression, epilepsy and chronic inflammatory diseases.

### LEGEND TO THE FIGURES

Figure 1: HPLC chromatogram of crude PEA
Figure 2: ¹H-NMR spectrum of crude PEA
Figure 3: HPLC chromatogram of purified PEA
Figure 4: ¹H-NMR spectrum of purified PEA

### EXPERIMENTAL SECTION

### Example 1: Analysis of impurities in crude PEA

Materials: crude PEA prepared by direct reaction of palmitic acid and ethanolamine was obtained from Zschimmer & Schwarz GmbH, Germany.

The crude preparation was analyzed using HPLC according to the following procedure:
Mobile phase A: mix 1.0 mL of formic acid with 1000 mL of Milli-Q.
Mobile phase B: mix 1.0 mL of formic acid with 1000 mL of ACN.
Column: Water Xbridge C18 5µm 250x3.0 mm (part number: 186003133)
Column temperature: 40 °C
Autosampler temperature: 18 °C
Injection volume: 20 µL
Detection: UV 200 nm

Gradient elution:

| Time Flow | Mobile phase A | Mobile phase B | |
|---|---|---|---|
| 0,01 min. | 1.00 mL/min | 40 % | 60 % |
| 11,00 min. | 1.00 mL/min | 0 % | 100 % |
| 25,00 min. | 1.00 mL/min | 0 % | 100 % |
| 25,01 min. | 1.00 mL/min | 40 % | 60 % |
| 30.00 min. | 1.00 mL/min | 40 % | 60 % |

Test solution (0.5 mg/mL): weigh 25.0 mg of PEA and dissolve in 50.0 mL of MeOH. If necessary, use ultrasonication and gently warm the solution until all PEA is dissolved.

Reference solution A (0.5 mg/mL): Weigh 25.0 mg of reference and dissolve in 50.0 mL of MeOH. If necessary, use ultrasonication and gently warm the solution until all PEA is dissolved.

Inject blank MeOH, reference solution A and test solution.

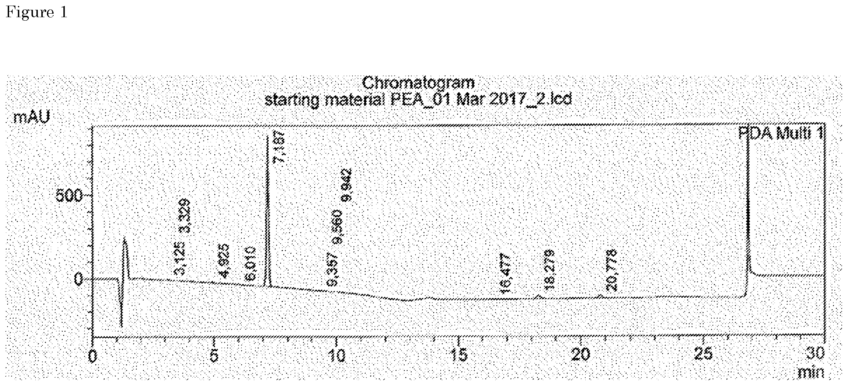

A representative HPLC chromatogram is given in Figure 1 and the proposed structures of impurities indicated therein are given in table 1. All impurities were confirmed by independent synthesis.

**Table 1 List of impurities**

| | |
|---|---|
| Impurity 1 | N-(2 -(2 -aminoethoxy)ethyl)palmitamide |
| Impurity 2 | Myristoylethanolamide |
| Impurity 3 | Stearoylethanolamide |
| Impurity 4 | *N,N'*-(oxybis(ethane-2,1-diyl))dipalmitamide |
| Impurity 5 | 2-Palmitamidoethyl palmitate |

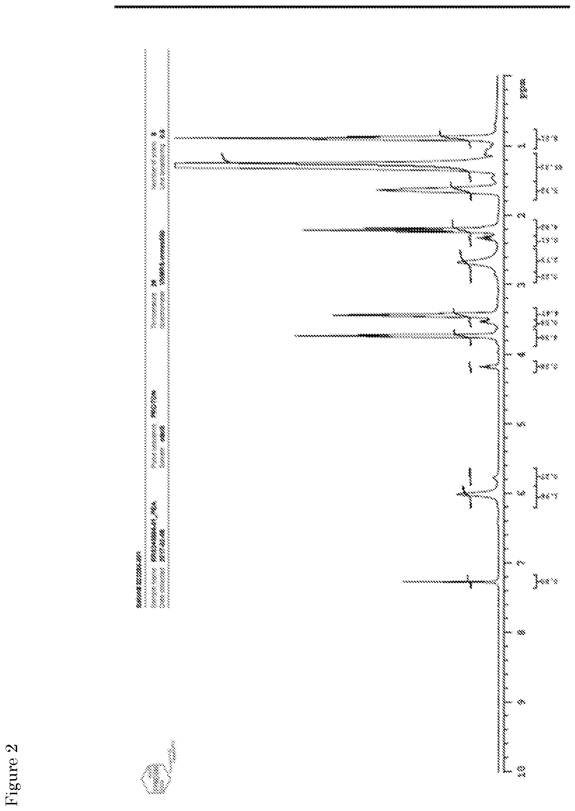

The ¹H-NMR spectrum in Figure 2 clearly shows the presence of impurities in crude PEA. The presence of myristoylethanolamide (cas # 142-58-5) and stearoylethanolamide (cas # 111-57-9) in the crud can be explained by the presence of myristic acid and stearic acid in the palmitic acid starting material. The presence of 2-palmitamidoethyl palmitate (PAEP) cas # 55349-67-2 can be explained by unwanted ester formation under the reaction conditions. The presence of *N,N'*-(oxybis(ethane-2,1-diyl))dipalmitamide (*N,N*-DPEA) and N-(2-(2-aminoethoxy)ethyl)palmitamide can be explained by the presence of 2,2'-oxybis(ethan-1-amine) in the crude ethanolamine starting material.

### Example 2: Purification of crude PEA using DBU

To a stirred suspension of crude PEA (1 kg, 3.34 mol) in a mixture of toluene (2.4 L) and methanol (1.6 L) 10 g DBU (1,8-Diazabicyclo[5.4.0]undec-7-ene 64 mmol (ca 20 mol %) was added. The reaction mixture was heated to reflux for 1 h and the mixture was allowed to cool overnight. The resulting white crystals were isolated over a Buchner funnel, washed with methanol and dried overnight at 35°C to give PEA as white crystals.

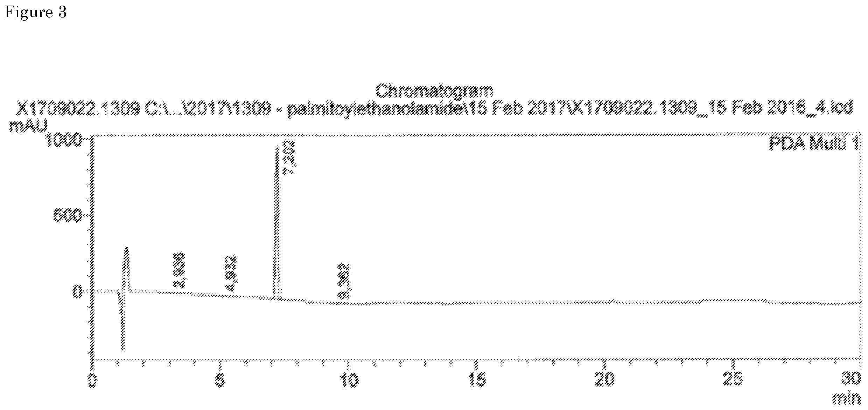

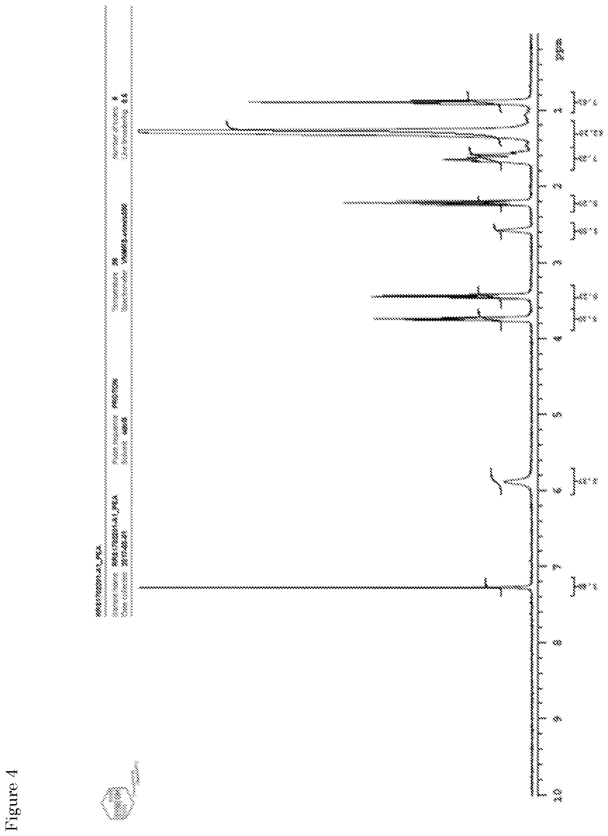

Figure 3 shows the HPLC chromatogram of purified PEA. Figure 4 shows the ¹H-NMR spectrum of purified PEA.

### Example 3: Large scale purification of crude PEA using DBU

To a stirred suspension of crude PEA (324 kg) in toluene (800 L) and methanol (500 L) was added DBU (5 kg). The reaction mixture was heated to reflux for 2.5 h and the mixture was allowed to cool overnight. The resulting white crystals were isolated over a Nutsche filter and dried using a floating bed dryer to give PEA as white crystals in ca. 75 % yield with an assay of > 99.5 %.

### Example 4 : Purification of crude PEA using NaHCO₃

To a stirred suspension of crude PEA (5 g) in toluene (12 mL) and methanol (8 mL) NaHCO₃ (100 mg) was added as base catalyst. The reaction mixture was heated to reflux for 1 h and the mixture was allowed to cool overnight. The resulting white crystals were isolated over a glass filter and dried overnight at 35°C to give PEA as white crystals in ca. 65 % yield.

### Example 5 : Purification of crude PEA using NaOMe

To a stirred suspension of crude PEA (50 g) in toluene (125 mL) and methanol (75 mL) was added NaOMe (135 mg). The reaction mixture was heated to reflux for 2 h and the mixture was allowed to cool overnight. The resulting white crystals were isolated over a glass filter and dried overnight at 35°C to give pure PEA as white crystals in ca 70 % yield.

### Example 6 : Purification of crude PEA using TBD

To a stirred suspension of crude PEA (50 g) in toluene (125 mL) and methanol (75 mL) was added TBD (450 mg). The reaction mixture was heated to reflux for 2 h and the mixture was allowed to cool overnight. The resulting white crystals were isolated over a glass filter and dried overnight at 35°C to give pure PEA as white crystals in ca 68 % yield.

## Claims

1. A method for providing pure palmitoyolethanolamide (PEA), comprising the steps of
(i) heating a suspension of crude PEA in a solvent mixture comprising an alcohol in the presence of a base catalyst having a pKA in water of at least 13 to reflux, thereby allowing the formation of crystalline PEA; and
(ii) isolating said crystalline PEA.

2. Method according to claim 1, wherein said crude PEA is obtained from a direct reaction of palmitic acid and ethanolamine.

3. Method according to claim 1 or 2, wherein said crude PEA comprises one or more unwanted substances selected from the group consisting of N-(2-(2-aminoethoxy)ethyl)palmitamide Myristoylethanolamide, Stearoylethanolamide, N,N'-(oxybis(ethane-2,1-diyl))dipalmitamide and 2-Palmitamidoethyl palmitate.

4. Method according to any one of the preceding claims, wherein the base catalyst has a pKa (in H₂O) of at least 13.5, preferably at least 14.

5. Method according to any one of the preceding claims, wherein the base catalyst is an amidine base, preferably selected from the group consisting of 1,8-diazabicyclo[5.4.0]undec-7-ene (DBU)); 1,5-diazabicyclo[4.3.0]non-5-ene (DBN), 2,3,4,6,7,8,9,10-octahydropyrimido[1,2-a]azepine), NaHCO₃ and NaOCH₃

6. Method according to any one of claims 1-4, wherein the base catalyst is a strong guanidine base, preferably triazabicyclodecene (1,5,7-Triazabicyclo[4.4.0]dec-5-ene (TBD).

7. Method according to any one of the preceding claims, wherein said solvent mixture comprises a C₁-C₃ alcohol, preferably methanol.

8. Method according to any one of the preceding claims, wherein said solvent mixture comprises or consists of an alcohol and toluene, preferably methanol and toluene.

9. Method according to claim 8, wherein said mixture consists of toluene and alcohol in a relative ratio in the range of 1.4 : 1 to 1.8 :1, preferably 1.5:1 to 1.7:1, by volume.

10. Method according to any one of the preceding claims, wherein said isolating comprises filtering and washing crystalline PEA.

## Patentansprüche

1. Verfahren zum Bereitstellen von reinem Palmitoyolethanolamid (PEA), umfassend die Schritte von
(i) Erhitzen einer Suspension von rohem PEA in einem Lösungsmittelgemisch, umfassend einen Alkohol, in der Gegenwart eines Basenkatalysators mit einem pKa in Wasser von wenigstens 13 bis zum Rückfluss, dadurch Ermöglichen der Bildung von kristallinem PEA; und
(ii) Isolieren des kristallinen PEA.

2. Verfahren nach Anspruch 1, wobei das rohe PEA aus einer direkten Reaktion von Palmitinsäure und Ethanolamin erhalten wird.

3. Verfahren nach Anspruch 1 oder 2, wobei das rohe PEA eine oder mehrere unerwünschte Substanzen umfasst, ausgewählt aus der Gruppe, bestehend aus N-(2-(2-Aminoethoxy)ethyl)palmitamid, Myristoylethanolamid, Stearoylethanolamid, N,N'-(Oxybis(ethan-2,1-diyl))dipalmitamid und 2-Palmitamidoethylpalmitat.

4. Verfahren nach einem der vorhergehenden Ansprüche, wobei der Basenkatalysator einen pKa (in H₂O) von wenigstens 13,5, vorzugsweise von wenigstens 14 hat.

5. Verfahren nach einem der vorhergehenden Ansprüche, wobei der Basenkatalysator eine Amidinbase ist, vorzugsweise ausgewählt aus der Gruppe bestehend aus 1,8-Diazabicyclo[5.4.0]undec-7-en (DBU)); 1,5-Diazabicyclo[4.3.0]non-5-en (DBN), 2,3,4,6,7,8,9,10-Octahydropyrimido[1,2-a]azepin), NaHCO₃ und NaOCH₃.

6. Verfahren nach einem der Ansprüche 1 - 4, wobei der Basenkatalysator eine starke Guanidinbase ist, vorzugsweise Triazabicyclodecen (1,5,7-Triazabicyclo[4.4.0]dec-5-en (TBD).

7. Verfahren nach einem der vorhergehenden Ansprüche, wobei das Lösungsmittelgemisch einen C₁-C₃-Alkohol, vorzugsweise Methanol, umfasst.

8. Verfahren nach einem der vorhergehenden Ansprüche, wobei das Lösungsmittelgemisch einen Alkohol und Toluol, vorzugsweise Methanol und Toluol, umfasst oder daraus besteht.

9. Verfahren nach Anspruch 8, wobei das Gemisch aus Toluol und Alkohol in einem relativen Verhältnis in dem Bereich von 1,4:1 bis 1,8:1, vorzugsweise 1,5:1 bis 1,7:1, nach Volumen besteht.

10. Verfahren nach einem der vorhergehenden Ansprüche, wobei das Isolieren Filtrieren und Waschen von kristallinem PEA umfasst.

## Revendications

1. Procédé pour obtenir du palmitoyléthanolamide (PEA) pur, comprenant les étapes suivantes :
(i) chauffage au reflux d'une suspension de PEA brut dans un mélange solvant comprenant un alcool en présence d'un catalyseur basique ayant un pKa dans l'eau d'au moins 13, ce qui permet ainsi la formation de PEA cristallin ; et
(ii) isolation dudit PEA cristallin.

2. Procédé selon la revendication 1, dans lequel ledit PEA brut est obtenu à partir d'une réaction directe d'acide palmitique et d'éthanolamine.

3. Procédé selon la revendication 1 ou 2, dans lequel ledit PEA brut comprend une ou plusieurs substances non souhaitées choisies dans le groupe constitué par le N-(2-(2-aminoéthoxy)éthyl)palmitamide, le myristoyléthanolamide, le stéaroyléthanolamide, le N,N'-(oxybis(éthane-2,1-diyl))dipalmitamide et le palmitate de 2-palmitamidoéthyle.

4. Procédé selon l'une quelconque des revendications précédentes, dans lequel le catalyseur basique a un pKa (dans H₂O) d'au moins 13,5, de préférence d'au moins 14.

5. Procédé selon l'une quelconque des revendications précédentes, dans lequel le catalyseur basique est une base de type amidine, de préférence choisie dans le groupe constitué par le 1,8-diazabicyclo[5.4.0]unéc-7-ène (DBU), le 1,5-diazabicyclo[4.3.0]non-5-ène (DBN), la 2,3,4,6,7,8,9,10-octahydropyrimido[1,2-a]azépine, NaHCO₃ et NaOCH₃.

6. Procédé selon l'une quelconque des revendications 1 à 4, dans lequel le catalyseur basique est une base forte de type guanidine, de préférence le triazabicyclodécène (1,5,7-triazabicyclo[4.4.0]déc-5-ène (TBD)).

7. Procédé selon l'une quelconque des revendications précédentes, dans lequel ledit mélange solvant comprend un alcool en C₁ à C₃, de préférence le méthanol.

8. Procédé selon l'une quelconque des revendications précédentes, dans lequel ledit mélange solvant comprend ou consiste en un alcool et du toluène, de préférence du méthanol et du toluène.

9. Procédé selon la revendication 8, dans lequel ledit mélange consiste en toluène et alcool en un rapport relatif situé dans la plage allant de 1,4/1 à 1,8/1, de préférence de 1,5/1 à 1,7/1, en volume.

10. Procédé selon l'une quelconque des revendications précédentes, dans lequel ladite isolation comprend une filtration et un lavage du PEA cristallin.
